# EUROPEAN PATENT SPECIFICATION

(11) **EP 2 911 651 B1**
(45) Date of publication and mention of the grant of the patent: **22.06.2016**
(21) Application number: 13762138.9
(22) Date of filing: 16.09.2013
(51) Int. Cl.: A61K 9/14, A61K 9/16, A61K 31/355

(54) **A STORAGE-STABLE DUST-FREE HOMOGENEOUS PARTICULATE FORMULATION COMPRISING AT LEAST ONE WATER-SOLUBLE VITAMIN E-DERIVATIVE AND AT LEAST ONE HYDROPHILIC POLYMER**
LAGERSTABILE, STAUBFREIE, HOMOGENE TEILCHENFÖRMIGE FORMULIERUNG MIT MINDESTENS EINEM WASSERLÖSLICHEN VITAMIN-E-DERIVAT UND MINDESTENS EINEM HYDROPHILEN POLYMER
FORMULATION PARTICULAIRE HOMOGÈNE SANS POUSSIÈRE STABLE EN STOCKAGE COMPRENANT AU MOINS UN DÉRIVÉ DE VITAMINE E HYDROSOLUBLE ET AU MOINS UN POLYMÈRE HYDROPHILE

(30) Priority: 27.09.2012 EP 12186326
(43) Date of publication of application: 02.09.2015
(73) Proprietor: BASF SE, 67056 Ludwigshafen am Rhein (DE)
(72) Inventor: GUTH, Felicitas, 67434 Neustadt (DE); KOLTER, Karl, 67117 Limburgerhof (DE); SCHÖNHERR, Michael, 67227 Frankenthal (DE); MÜLLER, Michael Klemens, 67454 Haßloch (DE)
(74) Representative: BASF IP Association
(86) International application number: PCT/EP2013/069111
(87) International publication number: WO 2014/048783

(56) References cited:
- EP-A1- 1 880 715
- WO-A1-2007/019058
- US-A- 4 603 143
- US-A- 5 891 469

## Description

The invention refers to a storage-stable dust-free homogeneous particulate formulation, consisting of at least one water-soluble Vitamin E-derivative, at least one hydrophilic polymer, optionally additional surface-active substances, and optionally additional pharmaceutical additives, with a fraction of fines passing through a sieve with a mesh size of 100 µm of less than 10 %-b.w.. In addition the invention refers to a process for manufacturing the formulation and the use as a solubilizing composition in pharmaceutical formulations.

It is well known in the art that surfactants can be used for enhancing the solubility of hydrophobic active ingredients in an aqueous medium and thus improve bioavailability of the active ingredient.

Among the various surfactants water-soluble vitamin E derivatives are known as potential agents for enhancing solubility. Well known water-soluble Vitamin E-derivatives are tocopheryl polyethylene glycol succinates, for instance a tocopheryl polyethylene 1000 succinate (TPGS). It is known for example from US 3,102,078 that TPGS can be used as a solubilizing agent for fat-soluble vitamins.

Due to their waxy nature tocopheryl polyethylene glycol succinates are difficult to handle. Many attempts have been made to overcome this disadvantage.

US 5,179,122 describes a solid composition where TPGS is absorbed or adsorbed to an inert carrier such as microcrystalline cellulose, starch or inorganic materials.

WO 01/00175 discloses mechanically stable pharmaceutical dosage forms which are solid solutions of active ingredients in an auxiliary agent matrix. The matrix contains a homopolymer or a copolymer of N-vinylpyrrolidone and a liquid or semi-solid surfactant.

WO 01/91727 discloses a self-emulsifying active substance formulation comprising at least one active substance and a formulation basis which includes a lipid component, a binder component and optionally additional auxiliary agents.

WO 00/57854 discloses mechanically stable pharmaceutical dosage forms comprising plastically mouldable, matrix-forming auxiliaries and more than 10 and up to 40% by weight of a surface-active substance with an HLB of between 2 and 18 that is liquid at 20°C, or has a drop point at between 20 and 50 °C. The auxiliaries are prepared by spray-drying or melt extrusion.

WO 2005/039551 discloses a solid pharmaceutical dosage form providing improved oral bioavailability for inhibitors of HIV protease. The dosage form comprises a solid dispersion of at least one HIV protease inhibitor and at least one pharmaceutically acceptable water-soluble polymer and at least one pharmaceutically acceptable surfactant, said pharmaceutically acceptable water-soluble polymer having a Tg of at least about 50 °C.

US 2005/0208082 discloses a solubilizing composition comprising a mixture of vitamin E TPGS and linoleic acid.

US 2005/0236236 discloses pharmaceutical compositions for administration of hydrophobic drugs comprising a hydrophobic drug, a vitamin E substance and a surfactant.

WO 2008/009689 discloses a solubilizing composition comprising a polyalkylene glycol derivative of a tocopheryl compound and at least one polyalkylene glycol fatty acid monoester or diester. The composition is obtained by melt-extrusion of the components.

WO 2009/130204 discloses solid compositions comprising permeability improving substances embedded in a water-soluble matrix. The compositions are obtained by normal spray-drying processes.

Known products still do not satisfy the requirements needed for safe and reliable manufacture of pharmaceutical formulations or dosage forms. Because of the tackiness of and relatively high amount of fines the material is not free-flowing tends to block dosage systems and other parts of the machinery. Another disadvantage of known materials is tendency to caking and therefore reduced storage stability. Yet another problem is phase separation of the waxy surfactant and the hydrophilic polymer, either during manufacture of the solubilizing composition or on storage.

The problem of the present invention was to provide a solubilizing composition based on water-soluble vitamin E-derivatives and hydrophilic polymers that is storage stable, dust-free, free of tackiness, free-flowing, easily miscible and offers good processability in the manufacture of pharmaceutical formulations. In addition, organic solvents should be avoided in manufacturing the solubilizing composition, not only because organic solvents are a safety risk, but also to avoid problems with the allowable residual solvents content.

The solution of this problem was to provide a storage-stable dust-free homogeneous particulate composition, consisting of at least one water-soluble Vitamin E-derivative, at least one hydrophilic polymer, optionally additional surface-active substances, and optionally additional pharmaceutical additives, with a fraction of fines of less than 10 % b.w. of particles with a diameter below 100 µm. the particulate formulation is obtained by a spray drying agglomeration process using aqueous spray solutions.

The inventive composition is a storage-stable dust-free particulate formulation, consisting of
(a) at least one water-soluble Vitamin E-derivative,
(b) at least one hydrophilic polymer,
(c) optionally additional surface-active substances, and
(d) optionally additional pharmaceutical additives,
with the proviso, that the sum of (a), (b), (c) und (d) equals 100 % by weight of the formulation, and wherein the fines fraction of the particles is less than 10 % by weight of particles with a diameter below 100 µm.

The inventive composition is a storage-stable dust-free particulate formulation, consisting of
(e) at least one water-soluble Vitamin E-derivative,
(f) at least one hydrophilic polymer,
(g) optionally additional surface-active substances, and
(h) optionally additional pharmaceutical additives,
with the proviso, that the sum of (a), (b), (c) und (d) equals 100 % by weight of the formulation, and wherein the fines fraction of the particles is less than 10 vol.- % of particles with a diameter below 100 µm, and wherein the average particle size of the particulate formulation as represented by the D_{0[3,4]} value ranges from 120 µm to 500 µm, preferably 180 to 350 µm, most preferably 200 to 300 µm. The average particle size is measured by laser diffraction and represents the volume average.

For all embodiments disclosed herein the bulk densities of the particulate formulations lie in the range of from 0.2 to 0.4 g/cm², preferably 0.25 -0.35 g/cm²

The angle of repose lies in the range of from 35 to 50 °, preferably 35 to 45 °. Component (a) is a water-soluble vitamin E-derivative, preferably a polyalkylene glycole derivative, most preferably tocopheryl polyethylene glycol succinates. Suitablepolyethylene glycol moieties are those with molecular weights of from 300 to 10.000 g/mol, for example PEG 300, PEG 400, PEG 1000, PEG 15000 or PEG 2000. Particularly preferred is a tocopheryl polyethylene glycol 1000 succinate, i.e. a product with a PEG 1000 moiety (TPGS 1000).

The water-soluble vitamin E derivative has a solubility of more than 10 % (g/g), preferably more than 25 % (g/g) at 20 °C.
TPGS 1000 has a solubility in water at 20 °C of more than 25 % (g/g) at normal pressure in the range of 0.1 MPa.
The amount of component (a) in the particulate formulation can range from 5 to 20 % by weight of the composition, preferably 10 to 20 % b.w..

Component (b) is selected from the group consisting of homo- or copolymers of an N-vinyl lactame, cellulose derivatives and polyacrylic polymers.

According to one preferred embodiment component (b) is a homo- or copolymer of N-vinyl pyrrolidone. Suitable polyvinyl pyrrolidone homopolymers show K values of from 12 to 100, preferably K 12 to 60 (1 % b.w. in water). According to this embodiment a most preferred component (b) is a copolymer of N-vinyl pyrrolidone and vinyl acetate, specifically a copolymer obtained from 6 parts of N-vinyl pyrrolidone and 4 parts of vinyl acetate (copovidone). The K value of such a most preferred copolymer lies in the range of from 20 to 40, particularly 25 to 31.

According to another embodiment component (b) is a copolymer of N-vinyl caprolactam, vinyl acetate and polyethylene glycol with K-values in the range of from 25 to 50, preferably in a weight ratio of 57 : 30 : 13 with a PEG 6000 moiety and with a K-value of 31 to 41 (1% b.w. solution in ethanol), commercially available as Soluplus®, BASF SE.

According to another preferred embodiment component (b) is a polyacrylate. According to this embodiment a most preferred component (b) is a copolymer of ethyl acrylate and methacrylic acid in a ratio of 1 : 1, commercially available as Kollicoat MAE 30 DP or 100P, or copolymers of methyl methacrylate and methacrylic acid in ratios of 1 : 1 and 2 : 1 commercially available as Eudragit L and S, or terpolymers of ethyl acrylate, methyl methacrylate and trimethylammonioethyl methacrylate in ratios of 1 : 2 : 0,2 or 1 : 2 : 0,1 commercially available as Eudragit RL and RS.

According to another preferred embodiment component (b) is a cellulose derivative, for example hydroxypropyl methyl cellulose acetat succinate (HPMCAS), hydroxypropyl methyl cellulose phthalate, celluloseacetate phthalate, celluloseacetate trimellitate, hydroxypropyl methyl cellulose (HPMC), hydroxypropyl cellulose, hydroxyethyl cellulose, methyl cellulose, preferably HPMCAS or HPMC.

According to another embodiment of the invention the particulate formulation optionally comprises a component (c) selected from the group consisting of polyalkylene glycol fatty acid esters, polyoxyalkylene glycol fatty alcohol ethers, polyoxyalkylene glycols, poloxamers, and polyoxyalkylene glycol glycerides. Another class of suitable components (c) are alkylene glycol fatty acid mono or diesters. Specific examples are polyoxyl 15 hydroxystearat, polyoxyl 40 hydrgogenated castor oil, polyethylene glycol with a molecular weight in the range from 300 to10 000, polyoxyethylene stearylether, polyoxyethylene laurylether, polyoxyethylene cetylether.

According to another embodiment the particulate composition optionally comprises a component (d) is selected from the group consisting of antioxidants, chelating agents, colorants, flavours, fillers, stabilizers, preservatives/ biocides. Suitable examples are natural or synthetic tocopherols, ascorbic acid, ethylenediamintetraacetic acid tetrasodium salt, silica, talc, magnesium stearate or butylated hydroxytoluene. A preferred biocide are silver ions. Other suitable preservatives are parahydroxy benzoic acid esters, benzoic acid salts, sorbic acid, benzalkonium chloride, Thiomersal, citric acid and its salts, propionic acid and its salts. Also ethanol or propylene glycol can be used as preservatives in concentrations of more than 15 % b.w. of the solution.

The particulate formulations according to the invention are consisting of
(a) 5 to 20 % b.w. of at least one water-soluble Vitamin E-derivative,
(b) 80 to 95 % b.w. of at least one hydrophilic polymer,
(c) 0 to 15 % b.w. of additional surface-active substances, and
(d) 0 to 15 % b.w. of additional pharmaceutical additives,
with the proviso that the total amount of (a), (b) and optionally (c) and/or (d) equals 100 % b.w. of the formulation, and wherein the fines fraction of the particles is less than 10 % b.w. of particles with a diameter below 100 µm.

Preferred particulate formulations are consisting of
(a) 10 to 20 % b.w. of at least one water-soluble Vitamin E-derivative,
(b) 80 to 90 % b.w. of at least one hydrophilic polymer,
(c) 0 to 15 % b.w. of additional surface-active substances, and
(d) 0 to 15 % b.w. of additional pharmaceutical additives. With the proviso, that the sum of (a), (b), (c) und (d) equals 100 % by weight of the formulation, and wherein the fines fraction of the particles is less than 10% b.w. of particles with a diameter below 100 µm.

According to one preferred embodiment particulate formulations are consisting of
(a) 10 to 20 % b.w. of at least one tocopheryl polyethylene glycol succinate (TPGS)
(b) 80 to 90 % b.w. of at least one hydrophilic polymer,
(c) 0 to 15 % b.w. of additional surface-active substances, and
(d) 0 to 15 % b.w. of additional pharmaceutical additives,
with the proviso, that the sum of (a), (b), (c) und (d) equals 100 % by weight of th formulation, and wherein the fines fraction of the particles is less than 10 % b.w. of particles with a diameter below 100 µm. According to this embodiment TPGS 1000 is particularly preferred.

According to another preferred embodiment particulate formulations are consisting of
(a) 10 to 20 % b.w. of at least one TPGS,
(b) 80 to 90 % b.w. of a polyacrylate,
(c) 0 to 15 % b.w. of additional surface-active substances, and
(d) 0 to 15 % b.w. of additional pharmaceutical additives,
with the proviso, that the sum of (a), (b), (c) und (d) equals 100 % by weight of theformulation, and wherein the fines fraction of the particles is less than 10 % b.w. of particles with a diameter below 100 µm. TPGS is preferably TPGS 1000. A preferred component (b) is a copolymer of ethyl acrylate and methacrylic acid in a ratio of 1 : 1, or copolymers of methyl methacrylate and methacrylic acid in ratios of 1 : 1 and 2 : 1 or terpolymers of ethyl acrylate, methyl methacrylate and trimethylammonioethyl methacrylate in ratios of 1 : 2 : 0,2 or 1 : 2 : 0,1.

According to another preferred embodiment particulate formulations are consisting of
(a) 10 to 20 % b.w. of at least one TPGS,
(b) 80 to 90 % b.w. of a cellulose derivative,
(c) 0 to 15 % b.w. of additional surface-active substances, and
(d) 0 to 15 % b.w. of additional pharmaceutical additives,
with the proviso, that the sum of (a), (b), (c) und (d) equals 100 % by weight of theformulation, and wherein the fines fraction of the particles is less than 10 % b.w. of particles with a diameter below 100 µm. TPGS is preferably TPGS 1000.

According to another preferred embodiment particulate formulations are consisting of
(a) 10 to 20 % b.w. of at least one TPGS,
(b) 80 to 90 % b.w. of a copolymer of N-vinyl caprolactam, vinyl acetate and polyethylenglycol,
(c) 0 to 15 % b.w. of additional surface-active substances, and
(d) 0 to 15 % b.w. of additional pharmaceutical additives,
with the proviso, that the sum of (a), (b), (c) und (d) equals 100 % by weight of theformulation, and wherein the fines fraction of the particles is less than 10 %b.w.- % of particles with a diameter below 100 µm. TPGS is preferably TPGS 1000. Preferably component (b) is a copolymer of N-vinyl caprolactam, vinyl acetate and polyethylene glycol 6000 with K-values in the range of from 25 to 50, preferably in a weight ratio of 57 : 30 : 13 with a K-value of 31 to 41 (1 % b.w. solution in ethanol).

According to another preferred embodiment particulate formulations are consisting of
(a) 10 to 20 % b.w. of at least one TPGS,
(b) 80 to 90 % b.w. of a copolymer of N-vinyl pyrrolidone and vinyl acetate,
(c) 0 to 15 % b.w. of additional surface-active substances, and
(d) 0 to 15 % b.w. of additional pharmaceutical additives,
with the proviso, that the sum of (a), (b), (c) und (d) equals 100 % by weight of theformulation, and wherein the fines fraction of the particles is less than 10% b.w. of particles with a diameter below 100 µm. TPGS is preferably TPGS 1000.

A particularly preferred embodiment relates to a particulate formulation consisting of
(a) 10 to 15 % b.w. of at least one TPGS,
(b) 85 to 90 % b.w. of a copolymer of N-Vinyl pyrrolidone and vinyl acetate,
(c) 0 to 15 % b.w. of additional surface-active substances, and
(d) 0 to 15 % b.w. of additional pharmaceutical additives,

With the proviso, that the sum of (a), (b), (c) und (d) equals 100 % by weight of the formulation, and wherein the fines fraction of the particles is less than 10 % b.w. of particles with a diameter below 100 µm. According to this preferred embodiment component (b) preferably is obtained from 6 parts of N-vinyl pyrrolidone and 4 parts of vinyl acetate. TPGS is preferably TPGS 1000.

Independently of which of the above described embodiments is chosen for the inventive particulate formulations, the formulations do not comprise a pharmaceutically active ingredient. The vitamin E derivative used according to the invention is used as a surfactant, but it can also serve as an active ingredient. Insofar the statement that the particulate formulations "do not comprise pharmaceutically active ingredient" means no pharmaceuticallly active ingredient other than the water-soluble vitamin E derivative.

The inventive particulate formulation is not a physical mixture of the components, but a formulated composition wherein the components cannot be separated from each other by mechanical process, such as for instance sieving.

The inventive particulate formulations are obtained by a spray drying agglomeration process, characterized by the following steps:
(i) forming an aqueous solution of components (a), (b) and optionally (c) and (d),
(ii) atomizing said solution with the help of one or more spray nozzles, contacting the atomized solution with particulate material consisting of components (a), (b) and optionally (c) and (d), and recovery of the dried particulate material.

According to a preferred embodiment the spray drying agglomeration process for manufacturing a particulate formulation according to the invention is characterized by the following steps:
(i) forming an aqueous solution of components (a), (b) and optionally (c) and (d),
(ii) atomizing said solution with the help of an atomizing device in a spray tower, contacting the atomized solution with fines of a particulate material consisting of components (a), (b) and optionally (c) and (d) and
(iii) separation of the fines and recirculation of the fines into the tower.

According to another preferred embodiment the spray drying agglomeration process for manufacturing a particulate formulation according to the invention is characterized by the following steps:
(i) forming an aqueous solution of components (a), (b) and optionally (c) and (d),
(ii) atomizing said solution with the help of an atomizing device in a spray tower, contacting the atomized solution with fines of a particulate material consisting of components (a), (b) and optionally (c) and (d) and
(iii) separation of fines and recirculation of the fines into the spray tower, and
(iv) drying of the particulate formulation in a fluidized bed.

### Step (i): Formation of the spray solution

Method (i) a): According to one embodiment of the invention step (i), i.e. the formation of the aqueous spray solution, is carried out in such a way that first of all component (a) is dissolved in water at elevated temperatures followed by blending in the solid hydrophilic polymer into the first solution. Optionally components (c) and (d) are also blended into the first solution. Elevated temperatures means 30 to 60 °C, preferably 37 to 55°C. Preferably the dissolution of the components is carried out under stirring.
Method (i) b): According to another embodiment the aqueous spray solution is prepared by dissolving the hydrophilic polymer at ambient temperatures in water and blending in component (a) in solid form. The blending in is preferably carried out under stirring. Optionally the blending in of component (a) can be carried out at elevated temperatures of from 30 to 60 ° C, preferably 37 to 55 °C.
Method (i) c): According to another embodiment component (a) is molten and blended into an aqueous solution of the hydrophilic polymer. The melting of component (a) can be carried out a 40 to 75 °C, preferably at 55 to 65 °C. Blending in the melt is preferably carried out under stirring.
Method (i) d): According to yet another embodiment a first solution of component (a) is formed at ambient temperatures or preferably at the above mentioned (Method (i) a) or b)) elevated temperatures. A second separate aqueous solution of the hydrophilic polymer is formed. The second solution can be formed at ambient temperatures. The resulting spray solution can be formed in a batch process either by blending in the first solution of component (a) into the second solution or by blending in the second solution into the first solution.
Method (i) e): The first and the second solution can also be blended in a continuous process, for instance by mixing a stream of the first solution with a stream of the second solution in a continuous mixing chamber.

Blending an aqueous solution of the hydrophilic polymer (b) into an aqueous solution of component (a) as described in connection with Method (i) d) is particularly preferred.

Independently of which of the Methods (i) a), b), c), d) or e) is used, the following conditions apply and can be combined where applicable:
The solids content of the spray solution can vary from 10 to 50 % b.w., preferably 20 to 40 % b.w., particularly 20 to 35 % b.w..
The concentration of the vitamin E derivative in the aqueous solution can range from 5 to 22 % b.w., preferably 8 to 19 % b.w. and most preferred 9 -17 % b.w..

The spray solution can be stored for several hours or overnight before being introduced into the spray drying agglomeration apparatus. The temperature of the stored solutions can be maintained in the range of from 37°C to 60°C, preferably from 40°C to 55°C and most preferably from 48 to 52°C. Most preferably the spray solution is kept stirring during the storage period.

Additional components such as the optional components (c) or (d) can be blended into the readymade spray solution comprising components (a) and (b) as such or in the form of an aqueous solution or dispersion.

### Step (ii): spray drying agglomeration

The inventive particulate formulation is obtained by a process known as spray drying-agglomeration. A spray drying -agglomeration process is characterized by a type of agglomeration known as a forced secondary agglomeration. A spray solution is atomized and agglomeration is controlled by returning fines to the atomized cloud. By definition fines are the cyclone or bag filter fractions or fractions separated by a particular air flow where they do not sediment anymore and consist of the smallest particles which are recycled to the process. According to Method (ii) a) the recycled dry fines are introduced into the spray tower near the atomizing nozzle(s) in the upper part of the spray apparatus where they will collide with the atomized droplets of the spray solution and thus form agglomerates. The particle diameter of such fines is preferably below 100 µm. The upper part of the spray apparatus means the upper 20 Vol.-% of the spray tower or spray apparatus. By introducing the fines near the atomizing devices such as nozzle(s or a rotating disc the contact between fines and atomized droplets is particularly enhanced.

Preferably method (ii) a) is combined with Step (iii), i.e. the recirculation of separated fines.

According to Method (iv) a fluidized bed can be operated at the bottom of the spray apparatus. The resulting particulate formulation can be subjected to further drying in such a fluidized bed.

According to a most preferred embodiment Methods (ii) a) and (iii) and (iv) are combined, particularly in combination with Method (i) d).

Particularly the invention relates to a spray drying agglomeration process for a particulate formulation consisting of
(a) 10 to 15 % b.w. of at least one TPGS,
(b) 85 to 90 % b.w. of a copolymer of N-Vinyl pyrrolidone and vinyl acetate,
(c) 0 to 15 % b.w. of additional surface-active substances, and
(d) 0 to 15 % b.w. of additional pharmaceutical additives,
with the proviso, that the sum of (a), (b), (c) und (d) equals 100 % by weight of the formulation, and wherein the fines fraction of the particles is less than 10 % b.w. of particles with a diameter below 100 µm, which process is characterized by a combination of Methods (i) d), (ii) a), (iii) and (iv).

According to this particular process embodiment component (b) preferably is obtained from 6 parts of N-vinyl pyrrolidone and 4 parts of vinyl acetate. TPGS is preferably TPGS 1000.

The resulting particles have a distinctive raspberry-like shape, sometimes also called blackberry-like shape.

Independently of which embodiment is used the following conditions for spray drying agglomeration apply:
The drying gas can be air, nitrogen or any other inert gas. Preferably air is used as drying gas. The drying gas is usually introduced at the top of the apparatus. According to one embodiment the major part of the drying gas is introduced at the top of the apparatus and the minor part of the drying gas is introduced into a fluidized bed located at the bottom of the apparatus. A major part of the drying gas can be 60 to 90 % b.w. of the gas. The amount of gas flow depends on the dimensions of the apparatus. The temperature of the drying gas usually lies in the range of 60 to 200 °C, preferably 85 to 130 °C for the inlet temperature at the top of the spray drying apparatus. The temperature of the drying gas which is introduced into the fluidized bed located at the bottom of the spray apparatus usually lies in the range of 20 to 80 °C, preferably 45 to 60 °C for the inlet temperature. The outlet temperature lies in the range of from 35 to 90 °C, preferably 45 to 70 °C.

Spraying is preferably performed with single component or two component nozzles. Alternatively, rotating discs can be used. Single component nozzles operate typically in the range of 2 -20 MPa atomizing pressure whereas two component nozzles use compressed air at 0.05 - 1 MPa.

According to a preferred embodiment the inventive process is carried out in such a way that a fluidized bed is operated at the bottom of the spray apparatus. The temperature within the fluidized bed is preferably kept below the melting temperature of component (a).

The amount of returned fines compared to spray velocity and gas flow depends on the dimensions of the apparatus.

The dried agglomerates are classified and the fines are returned into the apparatus.

The resulting inventive particulate formulation is stable in the packing on storage even at elevated temperatures such as 40 °C for at least three months.

Formulations according to any of the inventive embodiment can be tested for stability by measuring the firmness of the stored material in a water-tight packaging with a penetrometer. The penetrometer test measures the force necessary for pressing a cone under defined conditions into the store packaged material. This method can be used to control storage stability of a given packaged material.

After four weeks of storage at 40 °C and 10 % relative humidity the firmness is less than 1 N measured by 6 mm cone penetrometer. The firmness measured by using a 12 mm cone penetrometer is less than 3 N.

The inventive particulate formulation can be used to give pharmaceutical formulations or dosage forms by processing the particulate formulation together with one or more pharmaceutically active ingredients and optionally other excipients or additives.

One method for processing the inventive particulate formulation together with other ingredients to give a pharmaceutical formulation or dosage form is the melt-extrusion process. The melt-extrusion process comprises the steps of preparing a homogeneous melt of the active ingredient or the combination of active ingredients, the pharmaceutically acceptable polymer and the inventive particulate formulation, and cooling the melt until it solidifies. "Melting" means a transition into a liquid or rubbery state in which it is possible for one component to become homogeneously embedded in the other. Typically, one component will melt and the other components will dissolve in the melt, thus forming a solution. Melting usually involves heating above the softening point of the pharmaceutically acceptable polymer. The preparation of the melt can take place in a variety of ways. The mixing of the components can take place before, during or after the formation of the melt. For example, the components can be mixed first and then melted or simultaneously mixed and melted. Usually, the melt is homogenized in order to disperse the active ingredients efficiently. Also, it may be convenient first to melt the inventive particulate formulation and then to admix and homogenize the active ingredients.

Usually, the melt temperature (mass temperature) lies in the range of 100 to 350°C, preferably 100 to 300°C. If one or more lubricants is added the lower temperature can 50°C.

The active ingredients can be employed as such or as a solution or dispersion in a suitable solvent such as alcohols, aliphatic hydrocarbons or esters. Another solvent which can be used is liquid carbon dioxide. The solvent is removed, e.g. evaporated, upon preparation of the melt. Various additives may be included in the melt, for example flow regulators such as colloidal silica; lubricants, bulking agents (fillers), plasticizers, stabilizers such as antioxidants, light stabilizers, radical scavengers, or stabilizers against microbial attack. The melting and/or mixing takes place in an apparatus customary for this purpose. Particularly suitable are extruders or kneaders. Suitable extruders include single screw extruders, intermeshing screw extruders or else multi-screw extruders, preferably twin screw extruders, which can be co-rotating or counter-rotating and, optionally, equipped with kneading disks or other screw elements for mixing or dispersing the melt. The working temperatures will also be determined by the kind of extruder or the kind of configuration within the extruder used. Part of the energy needed to melt, mix and dissolve the components in the extruder can be provided by heating elements. However, the friction and shearing of the material in the extruder may also provide a substantial amount of energy to the mixture and aid in the formation of a homogeneous melt of the components. The melt is extruded through a die system. The extrudate leaving the extruder ranges from pasty to viscous. Before allowing the extrudate to solidify, the extrudate may be directly shaped into virtually any desired shape. Shaping of the extrudate may be conveniently carried out by a calender with two counter-rotating rollers with mutually matching depressions on their surface. Another option is to form films by calendering. Alternatively, the extrudate is moulded into the desired shape by injection-moulding. Alternatively, the extrudate is subjected to profile extrusion and cut into pieces, either before (hot-cut) or after solidification (cold-cut). Additionally, foams can be formed if the extrudate contains a propellant such as a gas, e.g. carbon dioxide, or a volatile compound, e.g. a low molecular-weight hydrocarbon, or a compound that is thermally decomposable to a gas. The propellant is dissolved in the extrudate under the relatively high pressure conditions within the extruder and, when the extrudate emerges from the extruder die, the pressure is suddenly released. Optionally, the resulting solid solution product is milled or ground to granules. The granules may then be filled into capsules or may be compacted. Compacting means a process whereby a powder mass comprising the granules is densified under high pressure in order to obtain a compact with low porosity, e.g. a tablet. Compression of the powder mass is usually done in a tablet press, more specifically in a steel die between two moving punches.

Alternatively the particulate formulation according to the invention can be formulated with other pharmaceutical ingredients by a melt-granulation process. In a melt granulation process the material is not converted to a homogeneous melt. The material is only heated to the extent that the surface of the inventive particulate formulation is molten so that the particles get tacky and start to adhere together. The melt granulation process can be carried out in a high-shear mixer or in an extruder which is operated without a die system. Since the extruder is in this case operated with an open discharge no pressure is build up within the extruder.

At least one additive selected from flow regulators, bulking agents (fillers) and lubricants is preferably used in compacting the granules.
Suitable bulking agents (also referred to as "fillers") are selected from lactose, calcium hydrogenphosphate, microcrystalline cellulose (Avicel®), magnesium oxide, potato or corn starch, isomalt, polyvinyl alcohol. Suitable flow regulators are selected from highly dispersed silica (Aerosil®), and animal or vegetable fats or waxes.
A lubricant is preferably used in compacting the granules. Suitable lubricants are selected from polyethylene glycol (e.g., having a Mw of from 1000 to 6000), magnesium and calcium stearates, sodium stearyl fumarate, talc and the like.

The inventive particulate formulations are particularly suitable for preparing pharmaceutical dosage forms comprising active ingredients with a solubility in water of less than 0.1%, preferably less than 0.01 % (g/g) at 20 °C and normal pressure.

The active ingredients may come from any range of indications.

Non-limiting examples which may be mentioned here are benzodiazepines, antihypertensives, vitamins, cytostatics - especially Taxol, anesthetics, neuroleptics, antidepressants, agents having antiviral activity, such as, for example, agents having anti-HIV activity, antibiotics, antimycotics, antidementia agents, fungicides, chemotherapeutics, urologicals, platelet aggregation inhibitors, sulfonamides, spasmolytics, hormones, immunoglobulins, sera, thyroid therapeutics, psychoactive drugs, antiparkinson agents and other antihyperkinetics, ophthalmologicals, neuropathy products, calcium metabolism regulators, muscle relaxants, anesthetics, lipid-lowering agents, hepatotherapeutics, coronary agents, cardiac agents, immunotherapeutics, regulatory peptides and their inhibitors, hypnotics, sedatives, gynecologicals, gout remedies, fibrinolytics, enzyme products and transport proteins, enzyme inhibitors, emetics, blood flow stimulators, diuretics, diagnostic aids, corticoids, cholinergics, biliary therapeutics, anti asthmatics, bronchodilators, beta-receptor blockers, calcium antagonists, ACE inhibitors, arteriosclerosis remedies, antiinflammatory drugs, anticoagulants, antihypertensives, antihypoglycemics, antihypertensives, antifibrinolytics, antiepileptics, antiemetics, antidotes, antidiabetics, antiarrhythmics, antianemics, antiallergics, anthelmintics, analgesics, analeptics, aldosterone antagonists, slimming agents.

The inventive particulate formulations can be used in a melt-extrusion process without showing any of the known disadvantages of prior art formulations. The particulate formulations are easily mixable with other compounds in particular with actives leading to so-called interactive mixtures which do not show segregation. Thus, a second feeder for the active is not necessary making the extrusion process easier. Surprisingly, the throughput rate of the extruder can be adjusted to extremely high values. Thus, on a 16 mm extruder at 200 rpm and a temperature of 150 °C more than 5.5 kg/h can be run, by far higher than the rates of the single components (Single components can be extruded on a 16 mm extruder at 200 rpm and a temperature of 160°C only with less than 4 kg/h).

Surprisingly, the inventive formulations do not lead to any dust formation. In addition they are characterized by an excellent flowability, which is maintained even when storing the material at elevated temperatures (40°C) for a long period of time (8 weeks). Usually, such soft materials and in particular materials with a small particle size of more than 10 % b.w. below 100µm show stickiness and cold flow behavior preventing it from a good flowability.
The inventive formulation and so called interactive mixtures thereof can be used in a direct compression process to manufacture tablets. Surprisingly, there is no sticking of the powder to the tablet punch which normally is likely to happen when physical mixtures with low melting substances are compressed.

The inventive formulation and so-called interactive mixtures of thereof can be used for roller compaction. Surprisingly it is possible to process the formulation in the compactor, even though a low melting substance is contained. Physical mixtures of the components cannot be processed because the low melting substance will stick to the rolls.
The inventive formulation and so-called interactive mixtures can be used in wet granulation processes (high shear granulation and fluid bed granulation). Surprisingly, the content uniformity of the granulated product can be achieved easily

The following methods can be used to characterize the physical properties of the particulate formulations.

### Particle Size

The analysis is performed with a Malvern Mastersizer 2000 Vers 5.22, Malvern Instruments, UK). The product is put on the sample plate (vibration intensity 100%) and dispersed with air a pressure of 0.05MPa. The measurement is carried out with an obscuration between 3-10%

### Bulk density:

The bulk density is the ratio of mass of an untapped powder sample to its volume. The powder is poured into a measuring cylinder of 150 ml and excess powder is discarded with a spatula. The mass of 150 ml Powder is obtained by differential weighing.

### Angle of repose:

The angle of repose is a characteristic related to resistance to movement between particles. It is the constant, three dimensional angle (relative to the horizontal base) assumed by a cone-like pile of material that is formed by draining excess quantity through a funnel by the method described below.
150 ml of untapped powder is filled into a measuring cylinder of 150 ml and excess powder is discarded with a spatula. The powder is poured into a funnel (Pfrengle type, diameter 1 cm), of which the opening is closed. The powder is drained from the funnel (if necessary under stirring with 1 rps) to a plate (diameter 10 cm, height 2,5 cm). The angle of repose is calculated from the relation of the height of the powder pile and the radius of the plate.

### Sieve analysis

The degree of fineness of a powder can be expressed by reference to a sieve.
The sieve analysis is performed with an Retsch AS 200 control sieving machine (100 g powder; amplitude: 1,0; sieving time: 10 min, without sieving aids or interruption). Separated fractions are determined by differential weighing.

### Example1:

### Formulation comprising 15 % b.w. TPGS 1000

62 kg water were heated to a temperature of 50°C. 9 kg molten TPGS (Temperature 60°C) are added under gentle stirring at low intensity (paddle, 250 rpm) and continuous heating (50°C) with a paddle stirrer. The stirrer was covered by the solution to avoid foaming. After 60 minutes of stirring TPGS was completely dissolved. 138 kg Copovidon solution with a solid content of 37 % b.w. were added under gentle stirring. The mixture was stirred for 15 minutes. The resulting solution was clear and transferred under continuous stirring to the dryer. The solids content was adjusted to 20 % b.w. by adding water.

**Spray drying agglomeration conditions:**

| | |
|---|---|
| Apparatus: | SBD dryer, Anhydro, height 2 m, diameter 1,2 m, two component nozzle; equipped with device for introducing fines and with a fluidized bed at the bottom |
| Solid content of the spray solution: | 20 % b.w. |
| Temperature of the gas, inlet tower: | 93°C |
| Temperature of the gas, fluidized bed: | 45°C |
| Amount of drying gas, inlet tower: | 450 kg/h |
| Amount of drying gas, fluidized bed: | 120 kg/h |
| Temperature exhaust air: | 50°C |
| Nozzle gas, pressure: | 0.15 MPa |
| Flow rate of solution : | 11,4 kg/h |

The particulate material was characterized as follows:
Angle of repose: 43°; bulk density: 0,33 g/cm³,
Not more than 10 % by weight of the sample passed the 100 µm sieve.
Particle sizes: d(0,1): 136µm; d(0,5):250 µm; d(0,9):426 µm; D[4,3]: 266 µm

### Example 2:

### Formulation comprising 10 % b.w. TPGS 1000

54 kg water were heated to a temperature of 50°C. 6 kg molten TPGS (Temperature 60°C) were added under gentle stirring (paddle, 250 rpm) and continuous heating (50°C) with a paddle stirrer. The stirrer was covered by the solution to avoid foaming. After 60 minutes of stirring TPGS was completely dissolved. 146 kg Copovidon Solution with a solid content of 37% are added under gentle stirring. The mixture was stirred for 15 minutes. The resulting solution was clear and transferred under continuous stirring to the spray apparatus.

**Spray drying agglomeration conditions:**

| | |
|---|---|
| Apparatus: | SBD dryer, Anhydro, height 2 m, diameter 1,2 m two component nozzle; equipped with device for introducing fines and with a fluidized bed at the bottom |
| Solid content of the solution: | 30 % b.w. |
| temperature of the gas, inlet tower: | 93°C |
| temperature of the gas, fluidized bed: | 45°C |
| Amount of drying gas, inlet tower: | 450 kg/h |
| Amount of drying gas, fluidized bed: | 120 kg/h |
| Temperature exhaust air: | 50°C |
| Nozzle gas, pressure: | 0.15 MPa |
| Flow rate of solution: | 11,4 kg/h |

The particulate material was characterized as follows:
Angle of repose: 44°; bulk density: 0,31g/cm³;
Particle sizes: d(0,1): 133 µm; d(0,5): 246 µm; d(0,9): 420µm D[4,3]: 262µm
Not more than 10 weight % of the sample passed the 100µm sieve.

### Comparative Example A:

### Preparation of a spray dried product

**A spray solution according to Example 1 was introduced into a spray drying apparatus with two component nozzles .The solution was sprayed under the following conditions:**

| Apparatus | Nubilosa spray tower, height 12m, diameter |
|---|---|
| | 0,8m |
| Solid content of the solution | 25 % b.w. |
| Temperature of the gas, inlet | 115°C |
| Temperature exhaust air | 55°C |
| Amount of drying gas | 450kg/h |
| Pressure of nozzle gas | 1,8bar |
| Flow rate of solution | 6,5kg/h |

Particle sizes: d(0,1): 30 µm; d(0,5): 83 µm; d(0,9): 168 µm D[4,3]: 93 µm
An angle of repose could not be measured due to the poor flowability of the product.

The firmness of the products according to examples 1 to 2 and Comparative Example A was determined with a cone penetrometer as described below.

### Test method penetrometer

The particulate material according to the invention was packed into aluminium foil laminnated polyethylene inliner bags (100 µm) of size DIN A 5. The bags were stored in a climate chamber with a relative humidity of 10 % either at 30 °C or 40 °C for 10 days, 4 weeks, 8 weeks. The bags were stored with a compression load of 2.2 kPa. After the pre-determined storage time the bags were opened in such a way that the compressed material remained undisturbed.

The firmness of the material was tested with a 6 mm or 12 mm cone tipped penetrometer by determining the force (in [N]) needed to insert the cone for 6 mm or 12 mm into the material.
The penetrometer used according to the examples was a digital mobile force meter PCE-FM 200, PCE Deutschland GmbH.

The results are depicted in the table below.

**Table I: Storage Stability, Cone Penetrometer Test, Firmness in [N]**

| | 10% TPGS | 15% TPGS | Comp.Ex. A |
|---|---|---|---|
| 10 days, 30°C, 6 mm cone | 0.07 | 0.15 | - |
| 10 day, 30 °C, 12 mm cone | 0.39 | 0.95 | - |
| 4 weeks, 30 °C, 6 mm cone [N] | 0.13 | 0.68 | - |
| 4 weeks, 40 °C, 6 mm cone [N] | 0.72 | 0.97 | 2.0 |
| 4 weeks, 30 °C, 12 mm cone [N] | 0.68 | 2.06 | - |
| 4 weeks, 40 °C, 12 mm cone [N] | 2.06 | 2.96 | 8.1 |
| 8 weeks, 30 °C, 6 mm cone | 0.23 | 0.73 | - |
| 8 weeks, 40 °C, 6 mm cone | 1.61 | 0.71 | 1.5 |
| 8 weeks, 30 °C, 12 mm cone | 0.86 | 1.35 | - |
| 8 weeks, 30 °C, 12 mm cone | 2.56 | 1.96 | 8.0 |

### Example 3

### Comparison of material throughput in a melt extrusion process

The product according to Example 1 was melt-extruded with a 16 mmThermofisher Polylab Extruder. For comparison, the single components, i.e. Kollidon VA 64 and TPGS 1000 were melt-extruded as well.
The product according to Ex. 1 could be extruded at 200 rpm and a temperature of 150 °C with a throughput of more than 5.5 kg/h. The single components could be extruded at 200 rpm and a temperature of 160°C only with a throughput less than 4 kg/h.

### Example 4:

### Formulation comprising 5 % b.w. TPGS 1000

45 kg water were heated to a temperature of 50°C. 3 kg molten TPGS (Temperature 60°C) were added under gentle stirring at low intensity (paddle, 250 rpm) and continuous heating (50°C) with a paddle stirrer. The stirrer was covered by the solution to avoid foaming. After 60 minutes of stirring TPGS was completely dissolved. 154 kg Copovidon solution with a solid content of 37 % b.w. were added under gentle stirring. The mixture was stirred for 15 minutes. The solids content was adjusted to 28% b.w. by adding water. The resulting solution was clear and transferred under continuous stirring to the dryer

**Spray drying agglomeration conditions:**

| | |
|---|---|
| Apparatus: | SBD dryer, Anhydro, height 2 m, diameter 1,2 m, two component nozzle; equipped with device for introducing fines and with a fluidized bed at the bottom |
| Solid content of the spray solution: | 28 % b.w. |
| Temperature of the gas, inlet tower: | 93°C |
| Temperature of the gas, fluidized bed: | 45°C |
| Amount of drying gas, inlet tower: | 450 kg/h |
| Amount of drying gas, fluidized bed: | 120 kg/h |
| Temperature exhaust air: | 50°C |
| Nozzle gas, pressure: | 0.15 MPa |
| Flow rate of solution : | 11,4 kg/h |

The particulate material was characterized as follows:
Angle of repose: 44°; bulk density: 0,30 g/cm³,
Not more than 10 % by weight of the sample passed the 100 µm sieve.
Particle sizes: d(0,1): 110µm; d(0,5):237 µm; d(0,9):405 µm; D[4,3]: 216 µm

### Example 5:

### Formulation comprising 20 % b.w. TPGS 1000

70 kg water was heated to a temperature of 50°C. 12 kg molten TPGS (Temperature 60°C) were added under gentle stirring at low intensity (paddle, 250 rpm) and continuous heating (50°C) with a paddle stirrer. The stirrer was covered by the solution to avoid foaming. After 60 minutes of stirring TPGS was completely dissolved. 130 kg Copovidon solution with a solid content of 37 % b.w. were added under gentle stirring. The mixture was stirred for 15 minutes. The solids content was adjusted to 25 %b.w. by adding water. The resulting solution was clear and transferred under continuous stirring to the dryer

**Spray drying agglomeration conditions:**

| | |
|---|---|
| Apparatus: | SBD dryer, Anhydro, height 2 m, diameter 1,2 m, two component nozzle; equipped with device for introducing fines and with a fluidized bed at the bottom |
| Solid content of the spray solution: | 25 % b.w. |
| Temperature of the gas, inlet tower: | 90°C |
| Temperature of the gas, fluidized bed: | 43°C |
| Amount of drying gas, inlet tower: | 450 kg/h |
| Amount of drying gas, fluidized bed: | 120 kg/h |
| Temperature exhaust air: | 50°C |
| Nozzle gas, pressure: | 0.15 MPa |
| Flow rate of solution : | 11,4 kg/h |

The particulate material was characterized as follows:
Angle of repose: 45°; bulk density: 0,35 g/cm³,
Not more than 10 % by weight of the sample passed the 100 µm sieve.
Particle sizes: d(0,1): 123µm; d(0,5):260 µm; d(0,9):425 µm; D[4,3]: 248 µm

The particulate material was tested for storage stability according to the cone penetrometer test as described above. The results are depicted in the table below. The product with 5% b.w. of TPGS was not tested, because the firmness of the material is influenced by the TPGS content in the formulation. A product with only 5% b.w. TPGS is at least as stable as a product with 10% b.w. TPGS.

**Table II: Storage Stability, Cone Penetrometer Test, Firmness in [N]**

| | Product with 20% TPGS |
|---|---|
| 10 days, 30°C, 6 mm cone | 0.25 |
| 10 day, 30 °C, 12 mm cone | 1.5 |
| 4 weeks, 30 °C, 6 mm cone [N] | 0.9 |
| 4 weeks, 40 °C, 6 mm cone [N] | 1.8 |
| 4 weeks, 30 °C, 12 mm cone [N] | 2.33 |
| 4 weeks, 40 °C, 12 mm cone [N] | 3.5 |
| 8 weeks, 30 °C, 6 mm cone | 2.57 |
| 8 weeks, 40 °C, 6 mm cone | 3.8 |
| 8 weeks, 30 °C, 12 mm cone | 3.1 |
| 8 weeks, 40 °C, 12 mm cone | 4.5 |

## Claims

1. A storage-stable dust-free homogeneous particle formulation, consisting of
(a) at least one water-soluble Vitamin E-derivative,
(b) at least one hydrophilic polymer,
(c) optionally additional surface-active substances, and
(d) optionally additional pharmaceutical additives,
with the proviso, that the sum of (a), (b), (c) und (d) equals 100 % by weight of the formulation, and wherein the fines fraction with particle diameters of less than 100µm is less than 10 % by weight.

2. Formulation according to Claim 1 with an average particle size D_{0[3,4]} of from 100 to 800 µm.

3. Formulation according to Claim 1 or 2, wherein component (a) is a tocopheryl polyethylenglycol succinate.

4. Formulation according to any of claims 1 to 3, wherein component (b) is selected from the group consisting of homo- or copolymers of an N-vinyl lactame, cellulose derivatives, poly acrylic polymers, polyalkylene oxids, polyvinyl alcohols and oligo- and polysaccharides.

5. Formulation according to any of claims 1 to 4, wherein component (b) is a homo-or copolymer of an N-vinyl lactame.

6. Formulation according to any of claims 1 to 5, wherein component (b) is a homo-or copolymer of N-vinyl pyrrolidone.

7. Formulation according to any of claims 1 to 6, wherein component (b) is a copolymer of N-vinyl pyrrolidone and vinyl acetate.

8. Formulation according to any of claims 1 to 7, wherein component (b) is a copolymer of N-vinyl caprolactam, vinyl acetate and polyethylene glycol.

9. Formulation according to any of claims 1 to 8, wherein component (b) is a cellulose derivative.

10. Formulation according to any of claims 1 to 9, wherein component (c) is selected from the group consisting of polyalkylene glycol fatty acid esters, polyalkylene glycol fattay alcohol ethers, polyalkylene glycols, poloxamers, polyalkylene glycol glycerides and alkylene glycol fatty acid mono- and diesters.

11. Formulation according to any of claims 1 to 10, wherein component (d) is selected from the group consisting of antioxidants, chelating agents, colorants, flavours, fillers, stabilizers, preservatives and biocides.

12. Formulation according to any of claims 1 to 11, wherein component (d) is ascorbic acid, tocopherol or butyl hydroxyl toluene.

13. Formulation according to any of claims 1 to 12, consisting of
(a) 5 to 20 % b.w. of at least one water-soluble Vitamin E-derivative,
(b) 80 to 95 % b.w. of at least one hydrophilic polymer,
(c) 0 to 15 % b.w. of additional surface-active substances, and
(d) 0 to 15 % b.w. of additional pharmaceutical additives.

14. Formulation according to anyof claims 1 to 13, consisting of
(a) 10 to 20 % b.w. of at least one water-soluble Vitamin E-derivative,
(b) 80 to 90 % b.w. of at least one hydrophilic polymer,
(c) 0 to 15 % b.w. of additional surface-active substances, and
(d) 0 to 15 % b.w. of additional pharmaceutical additives.

15. Process for manufacturing a particulate formulation according to any of Claims 1 to 14, **characterized by** the following steps:
(i) forming an aqueous solution of components (a), (b) and optionally (c) and (d),
(ii) atomizing said solution with the help of one or more spray nozzles, contacting the atomized solution with particulate material consisting of components (a), (b) and optionally (c) and (d).

16. Process according to Claim 15, wherein the particulate formulation is obtained by a spray drying agglomeration, **characterized by** the following steps:
(i) forming an aqueous solution of components (a), (b) and optionally (c) and (d),
(ii) atomizing said solution with the help of an atomizing device in a spray tower, contacting the atomized solution with fines of a particulate material consisting of components (a), (b) and optionally (c) and (d) and
(iii) separation of the fines and recirculation of the fines into the tower.

17. A process according to Claim15 or 16, **characterized by** the following steps:
(i) forming an aqueous solution of components (a), (b) and optionally (c) and (d),
(ii) atomizing said solution with the help of an atomizing device in a spray tower, contacting the atomized solution with fines of a particulate material consisting of components (a), (b) and optionally (c) and (d) and
(iii) separation of fines and recirculation of the fines into the spray tower, and
(iv) drying of the particulate formulation in a fluidized bed.

18. A process according to any of Claims 15 to 17, wherein the solids content of the spray solution can vary from 10 to 50 % b.w..

19. A process according to any of Claims 15 to 18, wherein step (i) is carried out by blending an aqueous solution of the hydrophilic polymer (b) into an aqueous solution of component (a).

## Patentansprüche

1. Lagerstabile staubfreie homogene Partikelformulierung, bestehend aus
(a) mindestens einem wasserlöslichen Vitamin-E-Derivat,
(b) mindestens einem hydrophilen Polymer,
(c) gegebenenfalls zusätzlichen oberflächenaktiven Substanzen und
(d) gegebenenfalls zusätzlichen pharmazeutischen Additiven,
mit der Maßgabe, dass die Summe von (a), (b), (c) und (d) gleich 100 Ges.-% der Formulierung ist, und wobei der Feingutanteil mit Teilchendurchmessern von weniger als 100 µm weniger als 10 Gew.-% beträgt.

2. Formulierung nach Anspruch 1 mit einer mittleren Teilchengröße D_{0[3,4]} von 100 bis 800 µm.

3. Formulierung nach Anspruch 1 oder 2, wobei es sich bei Komponente (a) um ein Tocopherylpolyethylenglykolsuccinat handelt.

4. Formulierung nach einem der Ansprüche 1 bis 3, wobei Komponente (b) aus der Gruppe bestehend aus Homo- oder Copolymeren eines N-Vinyllactams, Cellulosederivaten, Polyacrylpolymeren, Polyalkylenoxiden, Polyvinylalkoholen und Oligo- und Polysacchariden ausgewählt ist.

5. Formulierung nach einem der Ansprüche 1 bis 4, wobei es sich bei Komponente (b) um ein Homo- oder Copolymer eines N-Vinyllactams handelt.

6. Formulierung nach einem der Ansprüche 1 bis 5, wobei es sich bei Komponente (b) um ein Homo- oder Copolymer von N-Vinylpyrrolidon handelt.

7. Formulierung nach einem der Ansprüche 1 bis 6, wobei es sich bei Komponente (b) um ein Copolymer von N-Vinylpyrrolidon und Vinylacetat handelt.

8. Formulierung nach einem der Ansprüche 1 bis 7, wobei es sich bei Komponente (b) um ein Copolymer von N-Vinylcaprolactam, Vinylacetat und Polyethylenglykol handelt.

9. Formulierung nach einem der Ansprüche 1 bis 8, wobei es sich bei Komponente (b) um ein Cellulosederivat handelt.

10. Formulierung nach einem der Ansprüche 1 bis 9, wobei Komponente (c) aus der Gruppe bestehend aus Polyalkylenglykolfettsäureestern, Polyalkylenglykolfettalkoholethern, Polyalkylenglykolen, Poloxameren, Polyalkylenglykolglyceriden und Alkylenglykolfettsäuremonoestern und -diestern ausgewählt ist.

11. Formulierung nach einem der Ansprüche 1 bis 10, wobei Komponente (b) aus der Gruppe bestehend aus Antioxidantien, Chelatbildnern, Farbmitteln, Geschmacksstoffen, Füllstoffen, Stabilisatoren, Konservierungsstoffen und Bioziden ausgewählt ist.

12. Formulierung nach einem der Ansprüche 1 bis 11, wobei es sich bei Komponente (b) um Ascorbinsäure, Tocopherol oder Butylhydroxytoluol handelt.

13. Formulierung nach einem der Ansprüche 1 bis 12, bestehend aus
(a) 5 bis 20 Gew.-% mindestens eines wasserlöslichen Vitamin-E-Derivats,
(b) 80 bis 95 Gew.-% mindestens eines hydrophilen Polymers,
(c) 0 bis 15 Gew.-% zusätzlicher oberflächenaktiver Substanzen und
(d) 0 bis 15 Gew.-% zusätzlicher pharmazeutischer Additive.

14. Formulierung nach einem der Ansprüche 1 bis 13, bestehend aus
(a) 10 bis 20 Gew.-% mindestens eines wasserlöslichen Vitamin-E-Derivats,
(b) 80 bis 90 Gew.-% mindestens eines hydrophilen Polymers,
(c) 0 bis 15 Gew.-% zusätzlicher oberflächenaktiver Substanzen und
(d) 0 bis 15 Gew.-% zusätzlicher pharmazeutischer Additive.

15. Verfahren zur Herstellung einer partikelförmigen Formulierung nach einem der Ansprüche 1 bis 14, das durch folgende Schritte gekennzeichnet ist:
(i) Bilden einer wässrigen Lösung der Komponenten (a), (b) und gegebenenfalls (c) und (d),
(ii) Zerstäuben der Lösung mit Hilfe einer oder mehrerer Sprühdüsen, Kontaktieren der zerstäubten Lösung mit partikelförmigem Material, das aus den Komponenten (a), (b) und gegebenenfalls (c) und (d) besteht.

16. Verfahren nach Anspruch 15, wobei die partikelförmige Formulierung durch eine Sprühtrocknungsgglomeration erhalten wird, die durch folgende Schritte gekennzeichnet ist:
(i) Bilden einer wässrigen Lösung der Komponenten (a), (b) und gegebenenfalls (c) und (d),
(ii) Zerstäuben der Lösung mit Hilfe einer Zerstäubungsvorrichtung in einem Sprühturm, Kontaktieren der zerstäubten Lösung mit Feingut eines partikelförmigen Materials, das aus den Komponenten (a), (b) und gegebenenfalls (c) und (d) besteht, und
(iii) Abtrennen des Feinguts und Rezirkulieren des Feinguts in den Turm.

17. Verfahren nach Anspruch 15 oder 16, das durch folgende Schritte gekennzeichnet ist:
(i) Bilden einer wässrigen Lösung der Komponenten (a), (b) und gegebenenfalls (c) und (d),
(ii) Zerstäuben der Lösung mit Hilfe einer Zerstäubungsvorrichtung in einem Sprühturm, Kontaktieren der zerstäubten Lösung mit Feingut eines partikelförmigen Materials, das aus den Komponenten (a), (b) und gegebenenfalls (c) und (d) besteht, und
(iii) Abtrennen des Feinguts und Rezirkulieren des Feinguts in den Turm und
(iv) Trocknen der partikelförmigen Formulierung in einer Wirbelschicht.

18. Verfahren nach einem der Ansprüche 15 bis 17, bei dem der Feststoffgehalt der Sprühlösung von 10 bis 50 Gew.-% variieren kann.

19. Verfahren nach einem der Ansprüche 15 bis 18, bei dem Schritt (i) durch Einmischen einer wässrigen Lösung des hydrophilen Polymers (b) in eine wässrige Lösung von Komponente (a) durchgeführt wird.

## Revendications

1. Formulation particulaire homogène sans poussière stable au stockage, constituée par
(a) au moins un dérivé de vitamine E hydrosoluble,
(b) au moins un polymère hydrophile,
(c) éventuellement des substances tensioactives supplémentaires, et
(d) éventuellement des additifs pharmaceutiques supplémentaires,
à condition que la somme de (a), (b), (c) et (d) soit égale à 100 % en poids de la formulation, et dans laquelle la fraction de fines avec des diamètres de particules inférieurs à 100 µm représente moins de 10 % en poids.

2. Formulation selon la revendication 1 avec une taille moyenne de particules D_{0[3,4]} de 100 à 800 µm.

3. Formulation selon la revendication 1 ou 2, dans laquelle le composant (a) est un tocophérylsuccinate de polyéthylène glycol.

4. Formulation selon l'une quelconque des revendications 1 à 3, dans laquelle le composant (b) est choisi dans le groupe constitué par les homo- ou copolymères d'un N-vinyllactame, les dérivés de cellulose, les polymères polyacryliques, les oxydes de polyalkylène, les alcools polyvinyliques et les oligo-et polysaccharides.

5. Formulation selon l'une quelconque des revendications 1 à 4, dans laquelle le composant (b) est un homo- ou copolymère d'un N-vinyllactame.

6. Formulation selon l'une quelconque des revendications 1 à 5, dans laquelle le composant (b) est un homo- ou copolymère de N-vinylpyrrolidone.

7. Formulation selon l'une quelconque des revendications 1 à 6, dans laquelle le composant (b) est un copolymère de N-vinylpyrrolidone et d'acétate de vinyle.

8. Formulation selon l'une quelconque des revendications 1 à 7, dans laquelle le composant (b) est un copolymère de N-vinylcaprolactame, d'acétate de vinyle et de polyéthylène glycol.

9. Formulation selon l'une quelconque des revendications 1 à 8, dans laquelle le composant (b) est un dérivé de cellulose.

10. Formulation selon l'une quelconque des revendications 1 à 9, dans laquelle le composant (c) est choisi dans le groupe constitué par les esters d'acides gras de polyalkylène glycol, les éthers d'alcools gras de polyalkylène glycol, les polyalkylène glycols, les poloxamères, les glycérides de polyalkylène glycol et les mono- et diesters d'acides gras d'alkylène glycol.

11. Formulation selon l'une quelconque des revendications 1 à 10, dans laquelle le composant (d) est choisi dans le groupe constitué par les antioxydants, les chélateurs, les colorants, les arômes, les charges, les stabilisants, les conservateurs et les biocides.

12. Formulation selon l'une quelconque des revendications 1 à 11, dans laquelle le composant (d) est l'acide ascorbique, le tocophérol ou le butylhydroxytoluène.

13. Formulation selon l'une quelconque des revendications 1 à 12, constituée par
(a) 5 à 20 % en poids d'au moins un dérivé de vitamine E hydrosoluble,
(b) 80 à 95 % en poids d'au moins un polymère hydrophile,
(c) 0 à 15 % en poids de substances tensioactives supplémentaires, et
(d) 0 à 15 % en poids d'additifs pharmaceutiques supplémentaires.

14. Formulation selon l'une quelconque des revendications 1 à 13, constituée par
(a) 10 à 20 % en poids d'au moins un dérivé de vitamine E hydrosoluble,
(b) 80 à 90 % en poids d'au moins un polymère hydrophile,
(c) 0 à 15 % en poids de substances tensioactives supplémentaires, et
(d) 0 à 15 % en poids d'additifs pharmaceutiques supplémentaires.

15. Procédé de préparation d'une formulation particulaire selon l'une quelconque des revendications 1 à 14, **caractérisé par** les étapes suivantes :
(i) former une solution aqueuse des composants (a), (b) et éventuellement (c) et (d),
(ii) atomiser ladite solution à l'aide d'une ou plusieurs buses de pulvérisation, en mettant en contact la solution atomisée avec de la matière particulaire constituée par les composants (a), (b) et éventuellement (c) et (d).

16. Procédé selon la revendication 15, dans lequel la formulation particulaire est obtenue par une agglomération par séchage par atomisation, **caractérisé par** les étapes suivantes :
(i) former une solution aqueuse des composants (a), (b) et éventuellement (c) et (d),
(ii) atomiser ladite solution à l'aide d'un dispositif d'atomisation dans une tour de pulvérisation, en mettant en contact la solution atomisée avec des fines d'une matière particulaire constituée par les composants (a), (b) et éventuellement (c) et (d) et
(iii) séparer les fines et remettre les fines en circulation dans la tour.

17. Procédé selon la revendication 15 ou 16, **caractérisé par** les étapes suivantes :
(i) former une solution aqueuse des composants (a), (b) et éventuellement (c) et (d),
(ii) atomiser ladite solution à l'aide d'un dispositif d'atomisation dans une tour de pulvérisation, en mettant en contact la solution atomisée avec des fines d'une matière particulaire constituée par les composants (a), (b) et éventuellement (c) et (d) et
(iii) séparer les fines et remettre les fines en circulation dans la tour, et
(iv) sécher la formulation particulaire dans un lit fluidisé.

18. Procédé selon l'une quelconque des revendications 15 à 17, dans lequel la teneur en matières solides de la solution pulvérisée peut varier de 10 à 50 % en poids.

19. Procédé selon l'une quelconque des revendications 15 à 18, dans lequel l'étape (i) est réalisée en mélangeant une solution aqueuse du polymère hydrophile (b) dans une solution aqueuse du composant (a).
